# EUROPÄISCHE PATENTSCHRIFT

(11) **EP 2 050 392 B1**
(45) Veröffentlichungstag und Bekanntmachung des Hinweises auf die Patenterteilung: **05.09.2012**
(21) Anmeldenummer: 08011411.9
(22) Anmeldetag: 24.06.2008
(51) Int. Cl.: A61B 5/15, A61B 5/151

(54) **Lanzettenrad**
Lancet wheel
Roue à lancettes

(30) Priorität: 15.10.2007 EP 07020148
(43) Veröffentlichungstag der Anmeldung: 22.04.2009
(73) Patentinhaber: Roche Diagnostics GmbH, 68305 Mannheim (DE); F. Hoffmann-La Roche AG, 4070 Basel (CH)
(72) Erfinder: Hoenes, Joachim, 64673 Zwingenberg (DE); List, Hans, 64754 Hesseneck-Kailbach (DE); Zimmer, Volker, 67229 Laumersheim (DE); Hoerauf, Christian, 68723 Oftersheim (DE); Krämer, Uwe, 68549 Ilvesheim (DE)
(74) Vertreter: Mommer, Niels

(56) Entgegenhaltungen:
- EP-A- 1 190 674
- EP-A- 1 360 934
- EP-A- 1 402 812
- EP-A- 1 790 288
- EP-A- 1 880 671
- US-A1- 2005 277 850

## Beschreibung

Die Erfindung geht aus von einem Lanzettenrad mit den im Oberbegriff des Anspruchs 1 angegebenen Merkmalen. Ein derartiges Lanzettenrad ist aus der US 2005/0277850 A1 bekannt.

Derartige Lanzettenräder können als Lanzettenvorrat in ein Stechgerät eingesetzt werden, mit dem die Lanzetten des Lanzettenrades nacheinander verwendet werden, um zur Gewinnung einer Körperflüssigkeitsprobe eine Stichwunde in einem Körper eines Patienten zu erzeugen. Insbesondere für Diabetiker, die mehrmals täglich ihren Blutzuckergehalt überprüfen und dafür eine Körperflüssigkeitsprobe gewinnen müssen, ist es für einen möglichst hohen Benutzerkomfort wichtig, dass das mehr oder wenige aufwendig Einlegen eines frischen Lanzettenvorrats in das Stechgerät möglichst selten vorgenommen werden muss. Ein als Lanzettenrad ausgebildeter Lanzettenvorrat hat gegenüber Trommelmagazinen neben einer relativ kostengünstigen Herstellung insbesondere auch den Vorteil, dass sich eine größere Anzahl von Lanzetten Platz sparend anordnen lässt.

Aus der EP 1360934 A1 ist ein Lanzettenrad bekannt, bei dem die Spitzen der Lanzetten vor einem Stich aufgerichtet werden, indem sie aus der Radebene herausgeschwenkt werden. Dabei werden zwei sich senkrecht zur Längsrichtung der Lanzette verlaufende Stege tordiert.

Aus der WO 2007/060004 A1 ist es bekannt, bei einem Lanzettenrad durch Prägungen Knickbereiche vorzugeben. Wird die Lanzettenspitze eines solchen Lanzettenrades aus der Radebene herausgeschwenkt, gibt das Material in dem durch die Prägung geschwächten Bereich nach, so dass ein definierter Knick entsteht. Bei dem bekannten Lanzettenrad wird aber zum Schwenken der Lanzettenspitze und dem dafür erforderlichen Abknicken im Bereich der Lanzettenspitze eine Kraft auf die Lanzette ausgeübt. Ein Kontakt eines Geräteteils mit der Lanzettenspitze birgt jedoch die Gefahr einer Kontamination. Nachteilig ist auch, dass ein Lanzettenstich von Benutzern des bekannten Lanzettenrads häufig als schmerzhaft empfunden wird.

Aufgabe der Erfindung ist es, einen Weg aufzuzeigen, wie sich die bekannten Lanzettenräder verbessern lassen.

Diese Aufgabe wird durch ein Lanzettenrad mit den in Anspruch 1 angegebenen Merkmalen gelöst. Vorteilhafte Weiterbildungen der Erfindung sind Gegenstand der Unteransprüche. Die Aufgabe wird ferner durch ein Stichsystem mit einem derartigen Lanzettenrad gelöst.

Ein erfindungsgemäßes Lanzettenrad hat einen Träger, der die Lanzetten trägt, wobei die Lanzetten über Stege mit dem Träger verbunden sind, die eine Beweglichkeit der Lanzetten relativ zu dem Träger ermöglichen. Die Stege eines erfindungsgemäßen Lanzettenrads können dabei unterschiedliche Funktionen haben.

Eine erste Funktion der Stege eines erfindungsgemäßen Lanzettenrades besteht darin, das Aufrichten der Lanzette, also eine Schwenkbewegung retativ zu dem Träger, zu erleichtern. Hierfür genügt, dass sich zwischen einer Lanzette und einem benachbarten Teil des Trägers jeweils ein einziger Steg erstreckt. Ein solcher Steg lässt sich zum Aufrichten einer Lanzette mit einem wesentlichen geringeren Kraftaufwand biegen, als eine quer durch den Lanzettenkörper verlaufende Prägung, wie sie bei Lanzetten des aus der WO 2007/060004 A1 bekannten Lanzettenrads vorhanden ist. Insbesondere muss die Lanzette zum Aufrichten nicht im Bereich ihrer Spitze berührt werden, so dass die Gefahr einer Beeinträchtigung der Lanzettenspitze vermeiden werden kann.

Eine zweite mögliche Funktion der Stege eines erfindungsgemäßen Lanzettenrades besteht darin, für eine axiale Beweglichkeit der Lanzette relativ zu dem Träger zu sorgen und so einen schmerzärmeren Lanzettenstich zu ermöglichen. Ist nämlich eine Lanzette beidseitig über miteinander verkettete Stege mit dem Träger verbunden, kann eine Stechbewegung mit einer größeren Präzision ohne einen seitlichen Versatz, der zu unnötigem Schmerz führen kann, durchgeführt werden.

Bei bekannten Lanzettenrädern führt eine aufgerichtete Lanzette bei einem Stich eine Bewegung aus, die ähnlich wie bei einem Typenrad einer Schreibmaschine, entlang eines Kreisbogenabschnitts verläuft. Während eines Stichs kann dies im Körper eines Patienten zu einer lateralen Bewegung, also einem seitlichen Versatz, führen, der Schmerzen verursacht und die Stichwunde unnötig vergrößert. Durch miteinander verkettete Stege eines erfindungsgemäßen Lanzettenrades lässt sich eine geradlinige Stichbewegung verwirklichen, also einer solchen lateralen Bewegung entgegenwirken.

Bevorzugt sind jeweils nur zwei bis drei Stege miteinander verkettet. Dies hat den Vorteil, dass die axiale Beweglichkeit mit einem minimalen Herstellungsaufwand erreicht wird. Bevorzugt ist bei den miteinander verketteten Stegen jeweils ein Ende eines Stegs mit einem Ende eines benachbarten Stegs verbunden. Bei einem Stich stellt sich jeder der verketteten Stege, die bevorzugt mit ihren Längsseiten nebeneinander angeordnet sind, leicht schräg zur Ebene des Lanzettenrades, so dass sich eine zickzackförmige Konfiguration der miteinander verketteten Stege ergibt. Auf die se Weise wird eine Beweglichkeit der Lanzette in Stichrichtung, also senkrecht zur Ebene des Lanzettenrads, ermöglicht.

Bevorzugt ist der Träger ein Rahmen, der die Lanzetten des Lanzettenrades umgibt. Besonders bevorzugt ist dabei, dass die Lanzetten des Lanzettenrades jeweils allseitig von dem Rahmen umgeben sind. In einem Fenster des Rahmens können dabei eine oder mehrere Lanzetten angeordnet sein. Ein die Lanzetten umgebender Rahmen hat den Vorteil, die Stabilität des Lanzettenrades zu erhöhen.

Bevorzugt geht der Lanzettenkörper von einem Fuß aus, wobei die Stege die Lanzetten über den Fuß mit dem Träger verbinden. Der Fuß hat vorzugsweise eine andere Breite als der Lanzettenkörper. Erstrecken sich die Stege von dem Fuß ausgehend neben dem Lanzettenkörper, ist der Fuß bevorzugt breiter als der Lanzettenkörper. Der Fuß kann aber beispielsweise auch über einen einzigen Steg mit dem Träger verbunden sein, wobei sich der Steg in Längsrichtung des Lanzettenkörpers erstreckt. In einem solchen Fall kann der Fuß auch schmaler als der Lanzettenkörper sein.

Bevorzugt sind die Lanzetten eines erfindungsgemäßen Lanzettenrades in radialer Richtung ausgerichtet. Auf diese Weise lässt sich eine vorteilhaft Platz sparende Anordnung bewirken. Möglich ist es aber auch, die Lanzetten in einer anderen Orientierung anzuordnen.

Ein erfindungsgemäßes Lanzettenrad kann aus einer kreisförmigen Metallscheibe hergestellt werden, aus der durch Ausschneiden öder Ätzen Lanzetten, Stege und ganz oder teilweise auch der Träger gebildet werden. Neben Ausstanzen ist hierfür insbesondere das Laserschneiden geeignet.

Weitere Einzelheiten und Vorteile der Erfindung werden im Folgenden anhand von Ausführungsbeispielen unter Bezugnahme auf die beigefügten Zeichnungen erläutert. Gleiche und einander entsprechende Teile sind dabei mit übereinstimmenden Bezugszeichen versehen. Die im Rahmen der Ausführungsbeispiele beschriebenen Merkmale der Erfindung können einzeln oder in Kombination zum Gegenstand von Ansprüchen gemacht werden. Es zeigen:
- Figur 1: ein Ausführungsbeispiel eines Lanzettenrads;
- Figur 2a: eine schematische Darstellung eines Ausführungsbeispiels einer Lan- zette und der sie mit dem Rahmen eines Lanzettenrades verbindenden Stege;
- Figur 2b: eine Seitenansicht zu Figur 2a;
- Figur 3a: das in Figur 2a dargestellte Ausführungsbeispiel mit aufgerichteter Lan- zette;
- Figur 3b: eine Seitenansicht zu Figur 3a vor einem Stich;
- Figur 4: eine Seitenansicht gemäß Figur 3b während einem Stich;
- Figur 5: ein weiteres Ausführungsbeispiel einer Lanzette und der sie mit dem Rahmen eines Lanzettenrades verbindenden Stege;
- Figur 6: einen Ausschnitt eines weiteren Ausführungsbeispiels eines Lanzetten- rads; und
- Figur 7: ein Ausführungsbeispiel eines Stechsystems.

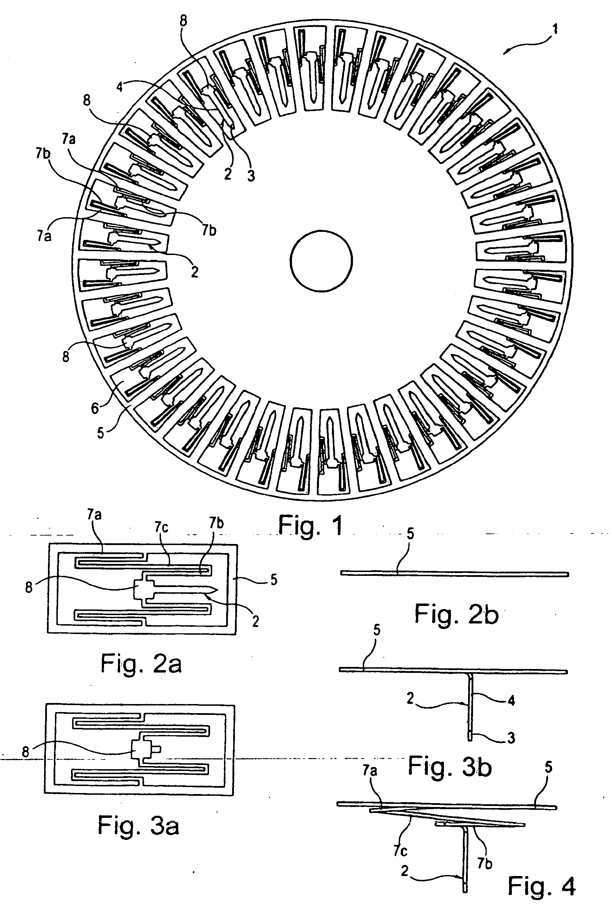

Figur 1 zeigt ein Ausführungsbeispiel eines Lanzettenrades 1 mit mehreren ringförmig angeordneten Lanzetten 2, die einen in einer Lanzettenspitze 3 endenden Lanzettenkörper 4 aufweisen, der von einem Fuß 8 ausgeht. Das Lanzettenrad 1 hat einen als Rahmen ausgebildeten Träger 5, der die Lanzetten 2 trägt. Bei dem dargestellten Ausführungsbeispiel ist der Rahmen 5 eine Kreisscheibe, die Aussparungen 6 als Fenster aufweist, in denen die Lanzetten 2 angeordnet sind. Die Lanzetten 2 sind über Stege 7a, 7b mit dem Rahmen 5 verbunden, die eine Beweglichkeit der Lanzetten 2 relativ zu dem Rahmen 5 ermöglichen.

Bei dem in Figur 1 dargestellten Ausführungsbeispiel sind die Lanzetten 2 jeweils beidseitig über jeweils zwei miteinander verkettete Stege 7a, 7b mit dem Rahmen 5 verbunden. Die Stege 7a, 7b sind schmaler als der Lanzettenkörper 4 und erstrecken sich jeweils in Längsrichtung der Lanzette 2, die von ihnen über ihren Fuß 8 mit dem Rahmen 5 verbunden ist. Einer der verketteten Stege 7a geht von dem Rahmen 5 aus, ein anderer Steg 7b geht von der Lanzette 2 aus. Die beiden verketteten Stege 7a, 7b sind mit ihren Längsseiten nebeneinander angeordnet und über jeweils eines ihrer Enden miteinander verbunden.

Figur 2a zeigt ein weiteres Ausführungsbeispiel zur Ausgestaltung der Lanzetten 2 und der sie mit dem Rahmen 5 verbindenden Stege 7. Bei dem in Figur 2a in einer Draufsicht dargestellten Ausführungsbeispiel ist die Lanzette 2 beidseitig über jeweils drei miteinander verkettete Stege 7a, 7b, 7c mit dem Lanzettenrahmen 5 verbunden, der in Figur 2a nur ausschnittsweise dargestellt ist und sich ähnlich wie bei dem in Figur 1 dargestellten Ausführungsbeispiel ringförmig erstreckt und eine Vielzahl von ringförmig angeordneten Lanzetten 2 trägt.

Das in Figur 2a ausschnittsweise dargestellte Lanzettenrad ist ebenso wie das in Figur 1 dargestellte Lanzettenrad aus einer Metallscheibe ausgeschnitten und hat deshalb die in Figur 2b gezeigte Seitenansicht. Vor einem Stich wird eine Lanzette 2 aufgerichtet, so dass sich die in Figur 3b gezeigte Seitenansicht und beispielsweise die in Figur 3a dargestellte Draufsicht ergibt.

Bei dem in Figur 3a dargestellten Ausführungsbeispiel ist der Lanzettenkörper 4 an seinem dem Fuß 8 zugewandten Ende abgebogen und die Lanzette 2 dadurch aufgerichtet. Um dieses Abbiegen des Lanzettenkörpers 4 zu erleichtern, kann der Lanzettenkörper 4 eine Prägung aufweisen, durch welche die Knickstelle definiert ist. Möglich ist es auch, zum Aufrichten der Lanzette 2 den Fuß 8 gegenüber dem Rahmen 5 zu verdrehen.

Während die Seitenansicht der Figur 3b die Verhältnisse vor einem Stich zeigt, sind in Figur 4 die Lanzette 2 und die sie mit dem Rahmen 5 verkettenden Stege 7a, 7b, 7c bei einem Stich dargestellt. Wie Figur 4 zeigt, neigen sich bei einem Stich die miteinander verketteten Stege 7a, 7b, 7c schräg gegenüber der Ebene des Rahmens 5.

Da die miteinander verketteten Stege 7a, 7b, 7c an ihren Enden verbunden sind, ergibt sich dadurch eine zickzackförmige Konfiguration, die eine Bewegung der Lanzette 2 in Stichrichtung ermöglicht.

Der große Vorteil der durch miteinander verkettete Stege 7a, 7b, 7c bewirkten axialen Beweglichkeit der Lanzetten 2 liegt darin, dass die Lanzette 2 bei einem Stich eine geradlinige Bewegung ausführen kann. Seitliche Bewegungen einer Lanzette 2 bei einem Stich führen nämlich zu Schmerz. Da seitliche Bewegungen, die sich insbesondere bei einer Stechbewegung entlang eines Kreisbogenabschnitts, ergeben würden, durch verkettete Stege 7a, 7b, 7c vermeiden werden können, lässt sich mit den vorstehend beschriebene Ausführungsbeispielen eine Körperflüssigkeitsprobe schmerzarm gewinnen.

Zum Ausführen eines Stichs kann eine Lanzette 2 beispielsweise durch einen Stößel eines nicht gezeigten Stechgeräts, in welches das Lanzettenrad 1 eingesetzt ist, in Stichrichtung bewegt werden. Ein derartiger Stößel kann an dem Fuß 8 einer Lanzetten 2 ansetzen, der bevorzugt breiter als der von ihm ausgehende Körper 4 der Lanzette 2 ist.

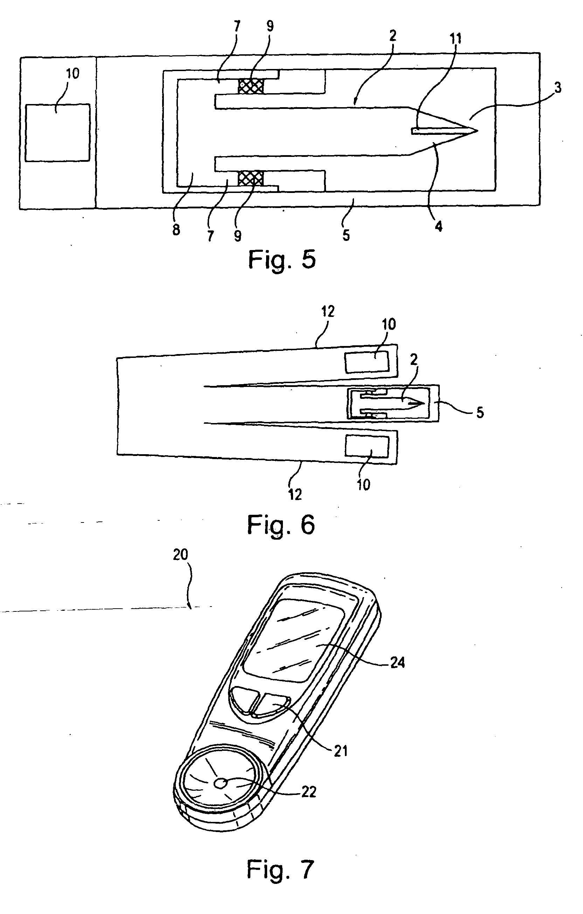

Figur 5 zeigt ein weiteres Ausführungsbeispiel einer über Stege 7 mit einem Lanzettenrahmen 5 verbundenen Lanzette 2. Ebenso wie in Figur 2a ist nur ein Ausschnitt eines Lanzettenrades dargestellt, bei dem sich der in Figur 5 dargestellte Ausschnitt ähnlich wie bei dem in Figur 1 gezeigten Lanzettenrad 1 ringförmig wiederholt.

Das in Figur 5 dargestellte Ausführungsbeispiel unterscheidet sich von den vorstehend beschriebenen Ausführungsbeispielen dadurch, dass die Lanzette 2 beidseitig jeweils nur über einen einzigen Steg 7 mit dem Rahmen 5 verbunden ist. Bei einem Aufrichten der Lanzette 2 biegen sich die Stege 7 in Knickstellen 9. Da die Stege 7 schmaler als der Lanzettenkörper 4 der Lanzette 2 sind, vorzugsweise weniger als halb so breit sind, ist hierfür nur eine geringe Kraft erforderlich. Um das Biegen weiter zu vereinfachen, können die Stege 7 durch Einprägungen oder anderer Schwächung vorbereitete Knickstellen 9 haben.

Ein Vorteil des in Figur 5 dargestellten Ausführungsbeispiels ist auch, dass sich die Lanzette 2 durch auf den Fuß 8 ausgeübten Druck aufrichten lässt. Dies ist mit einem geeigneten Stift oder Stempel in einem Stechgerät leicht möglich. Der vordere, der Lanzettenspitze 3 zugewandte Bereich des Lanzettenkörpers 4 wird dabei nicht berührt, so dass auch keine Gefahr einer Kontamination oder sonstigen Beeinträchtigung der Lanzettenspitze 3 besteht.

Bei dem in Figur 5 dargestellten Ausführungsbeispiel enthält die Lanzette 2 im Bereich ihrer Spitze 3 eine Probenaufnahmeeinrichtung 11, die beispielsweise als Kapillarspalt oder als Bohrung ausgeführt sein kann. Bei einem Stich füllt sich die Probenaufnahmeeinrichtung 11 mit einer Körperflüssigkeitsprobe. Zur Untersuchung der Körperflüssigkeitsprobe trägt der Rahmen 5 ein Testfeld 10. Das Testfeld 10 weist bevorzugt Nachweisreagenzien auf, die eine photometrische oder elektrochemische Bestimmung einer Analytkonzentration ermöglichen. Entsprechende Testfelder sind bei handelsüblichen Teststreifen, beispielsweise zur Blutzuckerbestimmung, vorhanden und bedürfen deshalb keiner weiteren Erläuterung.

Der Gebrauch der in Figur 5 dargestellten Lanzette vollzieht sich in den folgenden Schritten. Zunächst wird die Lanzette 2 aufgerichtet, beim dargestellten Ausführungsbeispiel also um einen Viertelkreisbogen gegenüber dem Rahmen 5 verschwenkt. Beim Aufrichten der Lanzette werden die Stege 7 im Bereich der Knickstellen 9 gebogen. Anschließend führt die Lanzette 2 eine schnelle Einstich- und Rückführbewegung aus, bei der die Probenaufnahmeeinrichtung 11 eine Körperflüssigkeitsprobe aufnimmt. Zur Untersuchung der aufgenommenen Körperflüssigkeitsprobe wird die Lanzette 2 anschließend nochmals gegenüber dem Rahmen 5 um einen Viertelkreisbogen geschwenkt, so dass die Probeneinrichtung 11 auf dem an dem Rahmen 5 angebrachten Testfeld 10 zu liegen kommt. Um das Übertragen der Körperflüssigkeitsprobe auf das Testfeld 10 zu erleichtern, hat dieses bevorzugt eine saugfähige Oberfläche, beispielsweise ein Vlies.

Das Testfeld 10 kann auf dem Rahmen 5 angeordnet sein. Bevorzugt ist das Testfeld 10 aber in einer Aussparung des Rahmens 5 angeordnet. Diese Maßnahme hat den Vorteil, dass eine Verfärbung des Testfeldes 10 photometrisch auf der von der Lanzette 2 gegenüberliegenden Seite der Rahmens 5 ausgewertet werden kann.

Die anhand der Figuren 1 bis 4 beschriebenen Ausführungsbeispiele können ebenfalls mit einem Testfeld 10 und die dazugehörenden Lanzetten 2 ebenso mit einer Probenaufnahmeeinrichtung 11 ausgestattet werden.

Um den Aufwand einer Probenaufnahmeeinrichtung 11 zu vermeiden, ist es auch möglich, Testfelder 10 auf dem Lanzettenrad 1 jeweils zwischen den einzelnen Lanzetten 2 auf Federarmen 12 anzuordnen. Figur 6 zeigt einen Ausschnitt eines Ausführungsbeispiels eines Lanzettenrades, bei dem zwischen den Lanzetten 2 jeweils Federarme 12, die an ihren freien Enden Testfelder 10 tragen, angeordnet sind. Eine Probenaufnahme erfolgt bei dem in Figur 6 dargestellten Ausführungsbeispiel dadurch, dass das Lanzettenrad 1 nach einem Stich soweit weitergedreht wird, dass ein Testfeld 10 über der zuvor erzeugten Stichwunde zu Liegen kommt. Anschließend wird das Testfeld 10 gegen die Stichwunde gepresst, so dass eine Probe aufgenommen wird.

Die vorstehend beschriebenen Ausführungsbeispiele von Lanzettenrädern 1 bilden mit einem dazu passenden Stechgerät ein Stechsystem. Ein derartiges Stechgerät hat eine Schwenkeinrichtung, um die Lanzetten 2 eines Lanzettenrades 1 für einen Stich aufzurichten, und einen Stechantrieb, um eine aufgerichtete Lanzette für einen Stich zu bewegen.

Figur 7 zeigt ein Ausführungsbeispiel eines derartigen Stechgeräts 20. Das Stechgerät 20 hat ein Gehäuse mit einer Gehäuseöffnung 22, an die ein Körperteil, in dem eine Stichwunde erzeugt wird, angelegt wird, Bedienungselemente 23 in Form von Tasten und eine Anzeigeeinrichtung 24 in Form einer Flüssigkristallanzeige zum Anzeigen von Untersuchungsergebnissen. Das Stechgerät 20 hat ein Aufnahmefach (nicht dargestellt), für ein Lanzettenrad 1. Das Aufnahmefach hat eine verschließbare Öffnung, die sich auf der Rückseite des in Figur 7 dargestellten Ausführungsbeispiels befindet.

In dem Stechgerät 20 sind zusätzlich zu der bereits erwähnten Schwenkeinrichtung und dem Stechantrieb auch eine Transporteinrichtung angeordnet, mit der ein in das Stechgerät 20 eingesetzten Lanzettenrad 1 gedreht werden kann, so dass dessen Lanzetten 2 nacheinander in eine Gebrauchsposition befördert werden, in der sie zum Erzeugen einer Stichwunde in einem an die Geräteöffnung 22 angelegten Körperteil verwendet werden können.

Um eine Kontamination der Lanzetten 2 eines Lanzettenrades 1 beim Einlegen in ein Stechgerät 20 auszuschließen, können die anhand der Figuren 1 bis 6 beschriebenen Lanzettenräder 1 beidseitig mit einer Sterilschutzfolie bedeckt sein. Beim Aufrichten einer Lanzette 2 wird die Sterilschutzfolie durchbrochen, so dass diese eine Stichbewegung nicht behindert. Die Sterilschutzfolien können mit Öffnungen für die Testfelder 10 versehen sein, um eine Probenaufnahme nicht zu beeinträchtigen. Möglich ist es auch ringförmige Sterilschutzfolien zu verwenden, welche die zu schützenden Lanzettenspitzen bedecken, die Testfelder aber unbedeckt lassen.

Nach einem Stich kann eine benutzte Lanzette 2 von dem Lanzettenrad 1 abgebrochen und von einem Benutzer entsorgt werden. Bevorzugt wird das Lanzettenrad 1 aber mit einer benutzten, noch aufgerichteten Lanzette 2 weitergedreht und die Lanzette 2 durch eine erneute Bewegung des Lanzettenrads 1 in Stichrichtung wieder in ihre Ausgangsposition zurück gebogen. Dazu kann an der Innenseite des Gehäuses 23 eine Schrägfläche angeordnet sein, auf die eine benutzte, aufgerichtete Lanzette 2 trifft und dadurch wieder zurück gebogen wird.

### Bezugszahlen

- 1: Lanzettenrad
- 2: Lanzetten
- 3: Lanzettenspitze
- 4: Lanzettenkörper
- 5: Rahmen
- 6: Aussparungen
- 7: Stege
- 7a: Stege
- 7b: Stege
- 7c: Stege
- 8: Fuß
- 9: Knickstellen
- 10: Testfeld
- 11: Probenaufnahmeeinrichtung
- 12: Federarme
- 20: Stechgerät
- 21: Bedienungselemente
- 22: Gehäuseöffnung
- 23: Gehäuse
- 24: Anzeigeneinrichtung

## Patentansprüche

1. Lanzettenrad mit mehreren ringförmig angeordneten Lanzetten (2), die einen in einer Lanzettenspitze (3) endenden Lanzettenkörper (4) aufweisen, und einem Träger (5), der die Lanzetten (2) trägt, wobei die Lanzetten (2) über Stege (7, 7a, 7b, 7c) mit dem Träger (5) verbunden sind, die eine Beweglichkeit der Lanzetten (2) relativ zu dem Träger (5) ermöglichen, und wobei sich die Stege (7, 7a, 7b, 7c) jeweils in Längsrichtung der Lanzette (2) erstrecken, die von ihnen mit dem Träger (5) verbunden ist, **dadurch gekennzeichnet, dass** die Lanzetten (2) zum Aufrichten relativ zu dem Träger (5) schwenkbar sind.

2. Lanzettenrad nach Anspruch 1, **dadurch gekennzeichnet, dass** der Träger ein Rahmen (5) ist, der die Lanzetten (2) umgibt.

3. Lanzettenrad nach einem der vorstehenden Ansprüche, **dadurch gekennzeichnet, dass** sich die Stege (7, 7a, 7b, 7c) zumindest teilweise entlang der Lanzette (2) erstrecken, die von ihnen mit dem Träger (5) verbunden ist.

4. Lanzettenrad nach einem der vorstehenden Ansprüche, **dadurch gekennzeichnet, dass** jede Lanzette (2) beidseitig über jeweils mindestens einen Steg (7, 7a, 7b, 7c) mit dem Träger (5) verbunden ist.

5. Lanzettenrad nach einem der vorstehenden Ansprüche, **dadurch gekennzeichnet, dass** die Lanzetten (2) beidseitig über miteinander verkettete Stege (7a, 7b, 7c) mit dem Träger (5) verbunden sind.

6. Lanzettenrad nach Anspruch 5, **dadurch gekennzeichnet, dass** die miteinander verketteten Stege (7a, 7b, 7c), die eine Lanzette (2) mit dem Träger (5) verbinden längs neben einander angeordnet sind.

7. Lanzettenrad nach Anspruch 5 oder 6, **dadurch gekennzeichnet, dass** sich zumindest einer der verketteten Stege (7a, 7b, 7c) entlang des Lanzettenkörpers (4) über dessen von der Lanzettenspitze (3) abgewandtes Ende hinaus erstreckt.

8. Lanzettenrad nach einem der vorstehenden Ansprüche, **dadurch gekennzeichnet, dass** die Stege (7, 7a, 7b, 7c) schmaler als die Lanzettenkörper (4) sind.

9. Lanzettenrad nach einem der vorstehenden Ansprüche, **dadurch gekennzeichnet, dass** der Lanzettenkörper (4) von einem Fuß (8) ausgeht, wobei die Stege (7, 7a, 7b, 7c) die Lanzetten (2) über den Fuß (8) mit dem Träger (5) verbinden.

10. Lanzettenrad nach einem der vorstehenden Ansprüche, **gekennzeichnet durch** Testfelder (10) zur Untersuchung einer Körperflüssigkeitsprobe.

11. Lanzettenrad nach Anspruch 10, **dadurch gekennzeichnet, dass** die Testfelder (10) von Federarmen (12) getragen werden, die zwischen den Lanzetten (2) angeordnet sind.

## Claims

1. Lancet wheel with a plurality of lancets (2) in a ring-shaped arrangement, said lancets (2) comprising a lancet body (4) that ends in a lancet tip (3) and a carrier (5) that carries the lancets (2), wherein the lancets (2) are connected to the carrier (5) via bars (7, 7a, 7b, 7c) that permit mobility of the lancets (2) relative to the carrier (5), and wherein the bars (7, 7a, 7b, 7c) each extend in the longitudinal direction of the lancet (2) they connect to the carrier (5), **characterized in that** the lancets (2) can be swiveled with respect to the carrier (5) for erecting them.

2. Lancet wheel according to claim 1, **characterized in that** the carrier is a frame (5) that surrounds the lancets (2).

3. Lancet wheel according to any one of the preceding claims, **characterized in that** the bars (7, 7a, 7b, 7c) extend, at least in part, along the lancet (2) they connect to the carrier (5).

4. Lancet wheel according to any one of the preceding claims, **characterized in that** each lancet (2) is connected on both sides to the carrier (5) via at least one bar (7, 7a, 7b, 7c).

5. Lancet wheel according to any one of the preceding claims, **characterized in that** the lancets (2) are connected on both sides to the carrier (5) via mutually concatenated bars (7a, 7b, 7c).

6. Lancet wheel according to claim 5, **characterized in that** the mutually concatenated bars (7a, 7b, 7c) connecting a lancet (2) to the carrier (5) are arranged longitudinally next to each other.

7. Lancet wheel according to claim 5 or 6, **characterized in that** at least one of the concatenated bars (7a, 7b, 7c) extends along the lancet body (4) beyond its end facing away from the lancet tip (3).

8. Lancet wheel according to any one of the preceding claims, **characterized in that** the bars (7, 7a, 7b, 7c) are narrower than the lancet body (4).

9. Lancet wheel according to any one of the preceding claims, **characterized in that** the lancet body (4) originates from a base (8), whereby the bars (7, 7a, 7b, 7c) connect the lancets (2) to the carrier (5) via the base (8).

10. Lancet wheel according to any one of the preceding claims, **characterized by** test fields (10) for analysis of a body fluid sample.

11. Lancet wheel according to claim 10, **characterized in that** the test fields (10) are carried by spring arms (12) that are arranged between the lancets (2).

## Revendications

1. Roue à lancettes comprenant plusieurs lancettes (2) disposées en forme de bague, qui présentent un corps de lancette (4) se terminant par une pointe de lancette (3) et un support (5), qui porte les lancettes (2), les lancettes (2) étant reliées au support (5) par des traverses (7, 7a, 7b, 7c), qui permettent une mobilité des lancettes (2) par rapport au support (5), et les traverses (7, 7a, 7b, 7c) s'étendant à chaque fois dans le sens longitudinal de la lancette (2), qui est reliée par les traverses au support (5), **caractérisée en ce que** les lancettes (2) peuvent pivoter par rapport au support (5) pour l'orientation.

2. Roue à lancettes selon la revendication 1, **caractérisée en ce que** le support est un cadre (5), qui entoure les lancettes (2).

3. Roue à lancettes selon l'une des revendications précédentes, **caractérisée en ce que** les traverses (7, 7a, 7b, 7c) s'étendent au moins en partie le long de la lancette (2) qui est reliée par elles au support (5).

4. Roue à lancettes selon l'une des revendications précédentes, **caractérisée en ce que** chaque lancette (2) est reliée des deux côtés au support (5) par respectivement au moins une traverse (7, 7a, 7b, 7c).

5. Roue à lancettes selon l'une des revendications précédentes, **caractérisée en ce que** les lancettes (2) sont reliées des deux côtés au support (5) par des traverses (7a, 7b, 7c) enchaînées les unes avec les autres.

6. Roue à lancettes selon la revendication 5, **caractérisée en ce que** les traverses (7a, 7b, 7c) enchaînées les unes avec les autres, qui relient une lancette (2) au support (5), sont disposées dans le sens longitudinal les unes à côté des autres.

7. Roue à lancettes selon la revendication 5 ou 6, **caractérisée en ce qu'**au moins l'une des traverses (7a, 7b, 7c) enchaînées s'étend le long du corps de lancette (4) au-delà de son extrémité opposée à la pointe de lancette (3).

8. Roue à lancettes selon l'une des revendications précédentes, **caractérisée en ce que** les traverses (7, 7a, 7b, 7c) sont plus étroites que les corps de lancette (4).

9. Roue à lancettes selon l'une des revendications précédentes, **caractérisée en ce que** le corps de lancette (4) part d'une base (8), les traverses (7, 7a, 7b, 7c) reliant les lancettes (2) au support (5) par l'intermédiaire de la base (8).

10. Roue à lancettes selon l'une des revendications précédentes, **caractérisée par** des zones de test (10) pour l'analyse d'un échantillon de liquide corporel.

11. Roue à lancettes selon la revendication 10, **caractérisée en ce que** les zones de test (10) sont portées par des bras à ressort (12) qui sont disposés entre les lancettes (2).
